# EUROPEAN PATENT APPLICATION

(11) **EP 2 727 562 A1**
(43) Date of publication of application: **07.05.2014**
(21) Application number: 13190335.3
(22) Date of filing: 25.10.2013
(51) Int. Cl.: A61F 2/95

(54) **Cannula attachment in endoluminal delivery devices**

(30) Priority: 31.10.2012 US 201261720848 P
(71) Applicant: Cook Medical Technologies LLC, Bloomington, IN 47404 (US)
(72) Inventor: Roeder, Blayne A., Bloomington, Indiana 47401 (US)
(74) Representative: Garratt, Peter Douglas

(57) **Abstract**

A stent graft delivery system in which a stent graft (6) is retained on a delivery device (2) such that a longitudinal portion of the stent graft is releasably retained on the delivery device to bend therewith. The delivery device (2) has a guide wire catheter (3) and the stent graft is retained onto the guide wire catheter by a cannula attachment wire (24) that can be withdrawn.

## Description

### FIELD OF INVENTION

This invention relates to a delivery device or deployment device for intraluminal or endovascular delivery of a stent graft.

### BACKGROUND OF THE INVENTION

It is important when delivering a stent graft by intraluminal or endovascular methods to know the exact location of the device in the vasculature particularly in relation to branch vessels which could be adversely occluded by stent graft placement. To avoid occlusion, fenestrations have been proposed along the length of a stent graft and scalloping at the proximal and distal ends of the stent graft to allow flow from a main vessel to a branch vessel through the fenestration or scallop.

The use of radiopaque markers around the periphery of the fenestration or scallop in this regard has been proposed, but it would be advantageous if a more certain or reliable method could be devised to assist with stent placement.

Particularly when deploying a graft into a curved vessel, it is known that a deployment device will take up a position in the vessel on the outside or greater radius side of the curved vessel. If a stent graft could be retained onto a deployment device so that a fenestration or scallop on the stent graft is in a selected position with respect to a portion of the deployment device when it is curved, then more accurate positioning may be possible.

As a particular example, the aorta of a patient comprises an ascending aorta from the aortic heart valve, a thoracic arch and a descending aorta. Major branch vessels extend from the thoracic arch and occlusion of one or more of these upon placement of a stent graft in the thoracic arch could have serious consequences. The major vessels generally extend from the outside or greater radius side of the curved thoracic arch. If a stent graft retained onto a deployment device such that a scallop on the stent graft was on the outside of the deployment device when it is curved, then correct placement would be more certain.

It is known to retain the proximal and distal ends of a stent graft onto a deployment device to facilitate relative longitudinal and rotational movement of the ends. Vessel tortuosity through which a deployment device must be progressed and the rotation necessary to achieve progression of the final rotational position of a stent graft, however, may not be fully known. In addition, in the process of positioning the stent graft, oftentimes attachment wires with the stent graft can become tangled with other objects in the deployment device.

It is an object of this invention to provide a retention mechanism for a stent graft onto a deployment device for more accurate deployment of the stent graft therefrom providing a practitioner with a useful alternative.

### SUMMARY OF THE INVENTION

In one aspect, an endovascular delivery device is disclosed, comprising: a nose cone dilator, a guide wire catheter having inner and outer surfaces, the inner surface defining a passageway with a longitudinal axis, and the outer surface having a plurality of openings radially located from the longitudinal axis; a stent graft comprising a tubular body of a biocompatible material with a lumen therethrough, the tubular body having first and second ends, and a plurality of stents, the stent graft being mounted onto the delivery device for deployment therefrom and being positioned on the guide wire catheter, passes through the lumen of the stent graft; a cannula attachment wire for releasably fastening a side of the stent graft to the guide wire catheter whereby the stent graft is temporarily affixed to the guide wire catheter along a length of the guide wire catheter defined by the length of the stent graft; wherein the cannula attachment wire weaves in and out of the plurality of openings.

In some embodiments, the guide wire catheter comprises at least three pairs of openings. In some embodiments, the cannula attachment wire and guide wire catheter conform a shape of the stent graft. In some embodiments, the cannula attachment wire weaves in and out of the stent graft. In some embodiments, the cannula attachment wire is substantially parallel to the longitudinal axis. In some embodiments, the plurality of openings are linearly arranged and substantially parallel to the longitudinal axis. In some embodiments, the delivery catheter comprises a longitudinal lumen therethrough, the guide wire catheter extending through the longitudinal lumen so that the guide wire catheter is movable longitudinally and rotationally with respect to the delivery catheter. In some embodiments, the endovascular delivery device also comprises a delivery catheter further comprising a flexible sheath over the delivery catheter and extending to the nose cone dilator and thereby retaining the stent graft in a contracted conformation on the guide wire catheter. In some embodiments, the endovascular delivery device further comprising a release mechanism operably connected to the cannula attachment wire.
In some embodiments, the guide wire catheter has a pre-formed curve located near the nose cone dilator. In some embodiments, the flexible sheath has a pre-formed curve located near the nose cone dilator.

In another aspect, an endovascular delivery device is disclosed, comprising: a delivery catheter; a nose cone dilator, a guide wire catheter comprising: a passageway with a longitudinal axis, a plurality of openings radially located from the longitudinal axis; a stent graft comprising: a tubular body of a biocompatible material with a lumen therethrough having first and second ends, a plurality of stents, wherein the stent graft is operably connected to the delivery device for deployment therefrom and being positioned on the guide wire catheter, wherein the guide wire catheter passes through the lumen of the stent graft; a cannula attachment wire operably connected to guide wire catheter and stent graft, wherein the cannula attachment wire weaves in and out of the plurality of openings.

In some embodiments, the endovascular device comprises at least three pairs of openings including one pair located substantially away from the first and second ends of the stent graft. In some embodiments, the cannula attachment wire and guide wire catheter conform a shape of the stent graft. In some embodiments, the cannula attachment wire weaves in and out of the stent graft. In some embodiments, the cannula attachment wire is substantially parallel to the longitudinal axis. In some embodiments, the plurality of openings is linearly arranged and substantially parallel to the longitudinal axis. In some embodiments, the delivery catheter comprises a lumen through which the guide wire catheter extends so that the guide wire catheter is movable longitudinally and rotationally with respect to the delivery catheter. In some embodiments, the delivery catheter comprises a flexible sheath located over the delivery catheter and extending to the nose cone dilator and thereby retaining the stent graft in a contracted conformation on the guide wire catheter; wherein the guide wire catheter has a pre-formed curve located near the nose cone dilator, and the flexible sheath has a pre-formed curve located near the nose cone dilator.

In another aspect, a method of deploying a stent graft to a patient in need thereof is disclosed, comprising: providing an endovascular delivery device, comprising: a nose cone dilator, a guide wire catheter having inner and outer surfaces, the inner surface defining a passageway with a longitudinal axis, and the outer surface having a plurality of openings radially located from the longitudinal axis; a stent graft comprising a tubular body of a biocompatible material with a lumen therethrough, the tubular body having first and second ends, and a plurality of stents, the stent graft being mounted onto the delivery device for deployment therefrom and being positioned on the guide wire catheter, which passes through the lumen of the stent graft; a cannula attachment wire for releasably fastening a side of the stent graft to the guide wire catheter whereby the stent graft is temporarily affixed to the guide wire catheter along a length of the guide wire catheter defined by the length of the stent graft; wherein the aligned openings substantially conform in shape to the cannula attachment wire; removing the cannula attachment wire.

### BRIEF DESCRIPTION OF THE DRAWINGS

Figure 1 depicts a introducer or delivery device according to one embodiment of the present invention.

Figures 2 depicts the introducer or delivery device of Figure 1 with the sheath withdrawn to show the components underneath it.

Figure 3A shows a longitudinal sectional view of a cannula attachment wire weaving in and out of a guide wire catheter.

Figure 3B shows a longitudinal sectional view of a cannula attachment wire weaving in and out of a stent graft and guide wire catheter.

Figures 4 shows a cross sectional view of a cannula attachment wire in a stent graft and guide wire catheter.

Figure 5 shows a perspective view of the embodiment shown in Figures 3A, 3B, and 4.

Figure 6 shows a schematic view of an aorta of a patient with a deployment device and stent graft deployed therein.

Figure 7 shows an alternative embodiment of an introducer or delivery device.

Figure 8 shows the embodiment of Figure 7 with the sheath withdrawn to show the components underneath that embodiment.

### DETAILED DESCRIPTION

Throughout this specification the term distal with respect to a portion of the aorta, a deployment device or a stent graft is intended to mean the portion of the aorta, deployment device or stent graft further away in the direction of blood flow away from the heart and the term proximal is intended to mean the portion of the aorta, deployment device or end of the stent graft nearer to the heart. When applied to other vessels similar terms such as caudal and cranial should be understood.

The invention will be discussed generally with respect to deployment of a stent graft into the thoracic aorta but is not so limited and may apply to deployment into other body lumens.

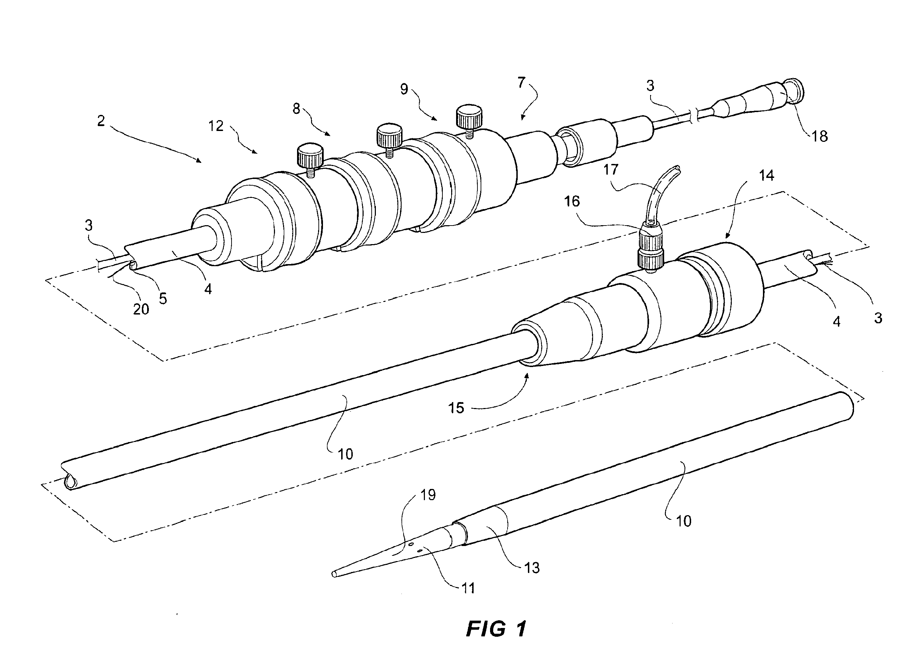

Figures **1-4** depict a delivery device **2.** The delivery device **2** has a guide wire catheter **3** which extends from a distal handle **7** to a proximal tapered nose cone dilator **11** longitudinally through a lumen **5** of a delivery catheter **4** which is connected to the handle **7** at its distal end. An introducer sheath **10** fits coaxially around the delivery catheter **4** and extends from a tapered proximal end **13** which optionally includes a radiopaque marker to a connector valve and manipulator **14** attached about distal end **15** of the introducer sheath **10.** The introducer sheath **10** extends proximally to the nose cone dilator **11** and covers the stent graft **6** during introduction of the deployment device into a patient and is withdrawn distally to expose the stent graft **6** during deployment when the deployment device is in a selected position within a patient's vasculature.

The stent graft or implantable device **6** is carried on the guide wire catheter **3** proximally of the delivery catheter **4** and distally of the nose cone dilator **11.** Connector valve **14** includes a silicone disk (not shown) for preventing the backflow of fluids therethrough. The disk includes a slit for the insertion of the nose cone dilator **11** and delivery catheter **4.** Connector **14** also includes side arm **16** to which a tube **17** is connected for introducing and aspirating fluids therethrough. Nose cone dilator **11** includes a tapered proximal end **19** for accessing and dilating a vascular access site over a well-known and commercially available wire guide (not shown).

The wire guide is inserted in the vessel with an introducer needle using, for example, a percutaneous vascular access Seldinger technique. A male Luer lock connector hub **18** is attached at the distal end of the guide wire catheter **3** for connection to syringes and other medical apparatus. The handle **7** at the distal end of the delivery catheter **4** remains outside a patient in use and carries the trigger wire release handle mechanisms **8, 9,** and **12** used to release the various portions of the stent graft. The proximal end the stent graft **6** is retained on the delivery device by the use of trigger wires (not shown) connected to the release device **9.** The distal end of the stent graft **6** is retained on the delivery device by the use of trigger wires (not shown) connected to the release device **8.** The handle also includes a release mechanism **12** for a cannula attachment wire for the longitudinal portion of the stent graft as discussed more below.

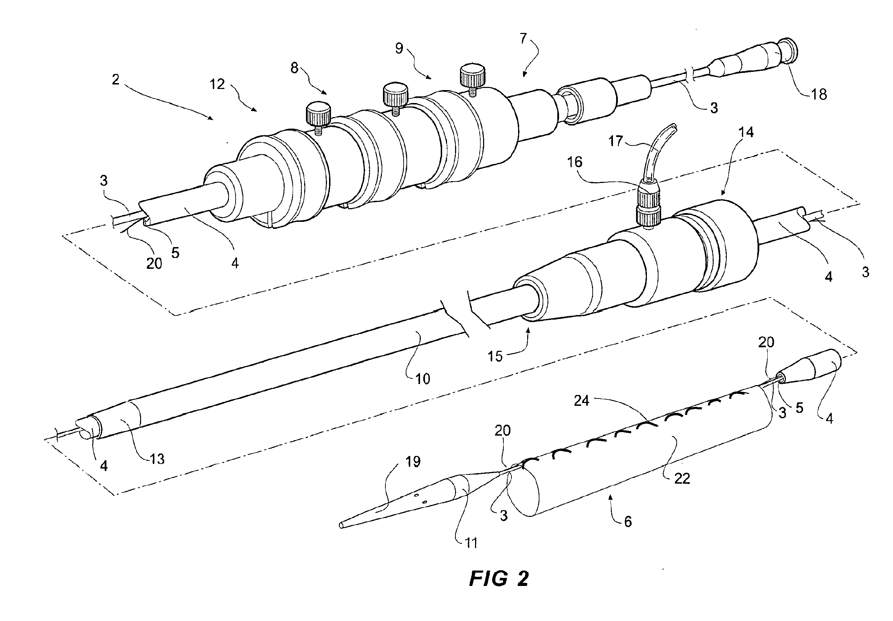

The stent graft **6** comprises a tubular body **22** of a biocompatible material and a plurality of self-expanding stents **27.** The tubular body has first and second openings **32** and **34.** A cannula attachment wire **20** extends from the release mechanism **12** on the handle **7** through the lumen **5** in the delivery catheter **4** and exits at the proximal end of the delivery catheter **4** as shown in Figure **2****.**

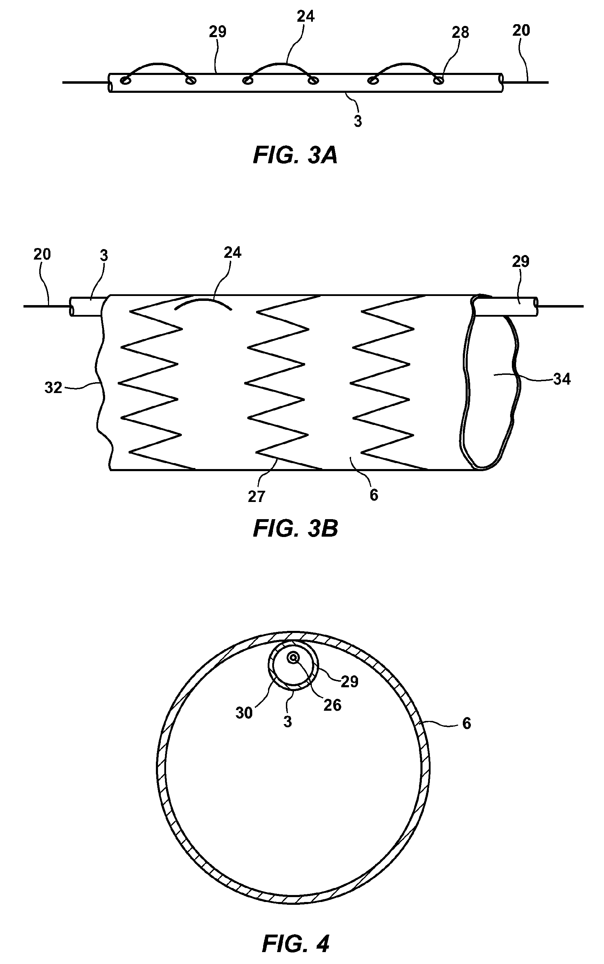

The cannula attachment wire **20** weaves through a plurality of openings **28** on the outer surface **29** of the guide wire catheter **3** and through the material of the tubular body **22** in an arrangement so that there are lengths **24** of cannula attachment wire **20** outside the tubular body **22** and, as can be particularly seen in Figures **3A, 3B,** and Figure **4****,** lengths **26** of cannula attachment wire **20** in the guide wire catheter **3.** The inner surface **30** of the catheter forms a passageway with a longitudinal axis. The plurality of openings **28** are radially located about the longitudinal axis.

In some embodiments, the plurality of openings **28** include aligned, paired openings through which the lengths **24** of cannula attachment wire **20** pass in and out of the guide wire catheter. In some embodiments, the cannula attachment wire passes through at three paired openings. In some embodiments, at least one pair of openings is located substantially away from the first and second openings **32** and **34** of the stent graft **6.** In some embodiments, the cannula attachment wire passes through as least three paired openings. In some embodiments, the alignment of the paired openings is linear such as that depicted in Figure **3A** and **3B****.**

These various arrangements hold the guide wire catheter **3** against a selected longitudinal portion of the stent graft. When the stent graft is deployed into the vasculature of a patient and the device is deployed into a curved vessel, the guide wire catheter of the deployment device will normally take up a position in the greater radius side of the curve owing to its relative rigidity. Hence the longitudinal portion of the stent graft which is releasably bound to the guide wire catheter will also take up a position at the greater radius side of the curve. This means that a stent graft with a fenestration or scallop which is to be deployed on the greater radius side of the curve can be mounted onto the deployment device with the fenestration or scallop adjacent the guide wire and once deployed the scallop or fenestration will be easier to correctly position.

When it is desired to release the stent graft, the cannula attachment wire **20** can be withdrawn by removal of the release wire mechanism **12** which will release the stent graft from its engagement with the guide wire catheter **3** as well as the stent graft **6.** The linear arraignment of the plurality of openings **28** ease removal of the cannula attachment wire **20** as compared to configurations where a wire is spirally disposed about stent graft and guide wire catheter.

In the depicted embodiment, the order of placement of the release handle mechanisms **8, 9,** and **12** on the handle of the introducer is such that the order of release is the proximal end of the stent graft, the distal end of the stent graft and then the longitudinal portion. In other embodiments and assemblies the order of placement may be different. For instance it may be desirable to release the proximal end, the longitudinal portion and then the distal end.

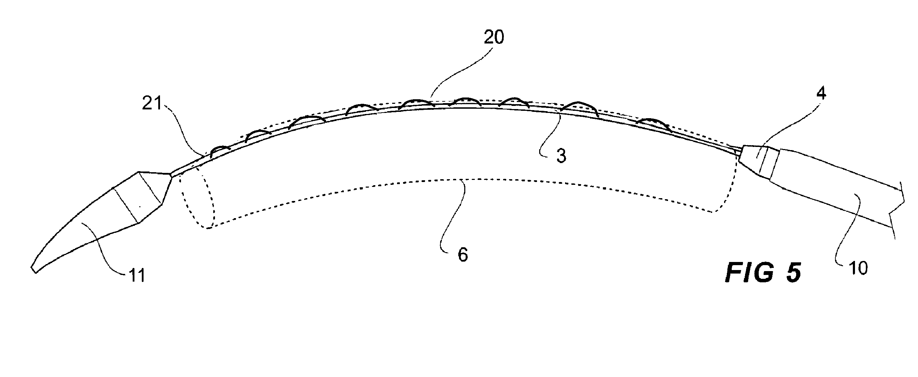

Figure **5** shows an embodiment when a guide wire catheter is curved such as when it is placed into a curved vessel such as the thoracic aorta of a patient. It will be noted that the stent graft **6** when not constrained by the sheath **10** has a longitudinal portion retained against the guide wire catheter **3** and otherwise extends out to one side of the guide wire catheter. The stent graft has a scallop **21** at its proximal end and the scallop is aligned with the guide wire catheter. When the guide wire catheter **3** is bent, the longitudinal portion of the stent graft with a scallop **21** is deployed on the greater radius side of the curve.

If by suitable imaging techniques the great vessel such as the left subclavian artery is seen to be off center at the top of the curve of the thoracic arch then the stent graft can be mounted onto the deployment device with its scallop similarly off center from the longitudinal portion aligned with the guide wire catheter of the deployment device.

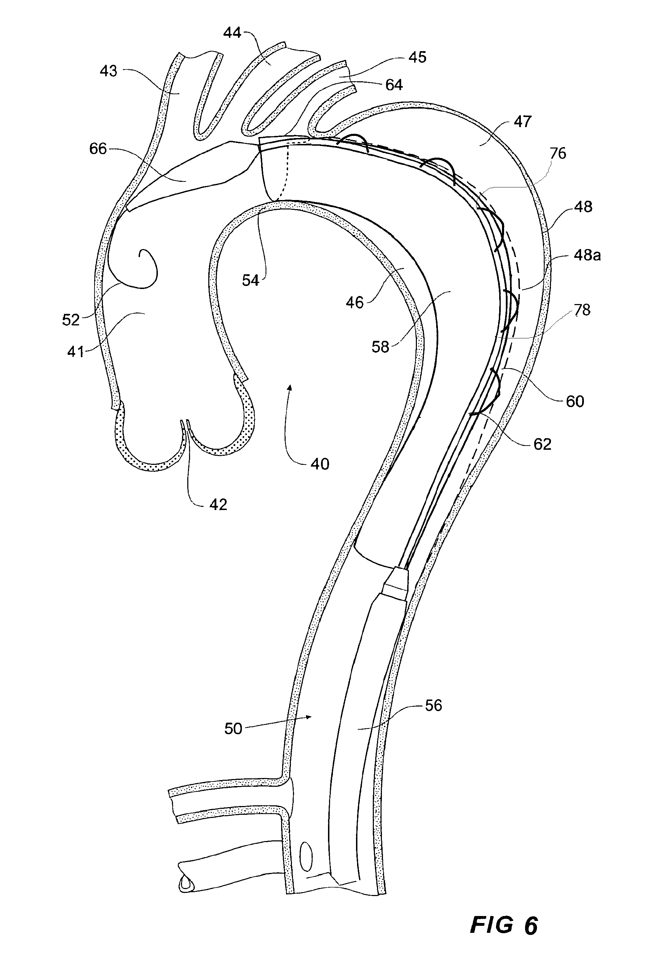

Figure **6** shows a cross sectional view of a thoracic aorta. It will be seen that the thoracic aorta **40** comprises an ascending aorta **41** which receives blood from the heart though an aortic valve **42.** At the upper end of the ascending aorta, there are branches for the great vessels, the innominate artery **43,** the left common carotid artery **44** and the left subclavian artery **45.** The aorta after these great vessels is referred to as the descending aorta **46** and it is in this region that a thoracic aortic aneurysm **47** can occur. In a thoracic aortic aneurysm part of the wall **48** of the descending aorta swells and can burst with serious consequences. The dotted line **48a** shows what would be the normal curved wall of the descending aorta.

As shown in Figure **6****,** a deployment device **50** has been deployed up through the descending aorta over a guide wire **52.** The proximal end of the deployment device extends over the thoracic arch **54** and into the ascending aorta **41.** The sheath **56** has been withdrawn to partially release the stent graft **58,** but it is still retained by the cannula attachment wire **60** onto the guide wire catheter **62** of the deployment device **50.** The cannula attachment wire **60** weaves linearly in and out of a plurality of openings **76** of an outer surface **78** of the guide wire catheter **62** and through the material of the stent graft **58.** It will be noted that as the thoracic arch is curved the guide wire catheter of the deployment device has taken up a position on the greater radius side of the curve and hence the longitudinal portion of the stent graft which is releasably bound to the guide wire catheter has also take up a position at the greater radius side of the curve. This enables the scallop **64** at the proximal end of the stent graft **58,** which is positioned adjacent the guide wire catheter **62,** to be adjacent the left subclavian artery **45** so that the stent graft does not occlude that artery. At the stage shown, both the proximal and distal ends of the stent graft have been released, but as discussed earlier, the distal end may be retained and not released until the longitudinal portion retention has been released.

Withdrawal of the cannula attachment wire **60** by removal of the release handle mechanism **12** (see Figure **1****)** enables the nose cone dilator **66** to be retracted to the sheath **56** and the entire deployment device can be withdrawn.

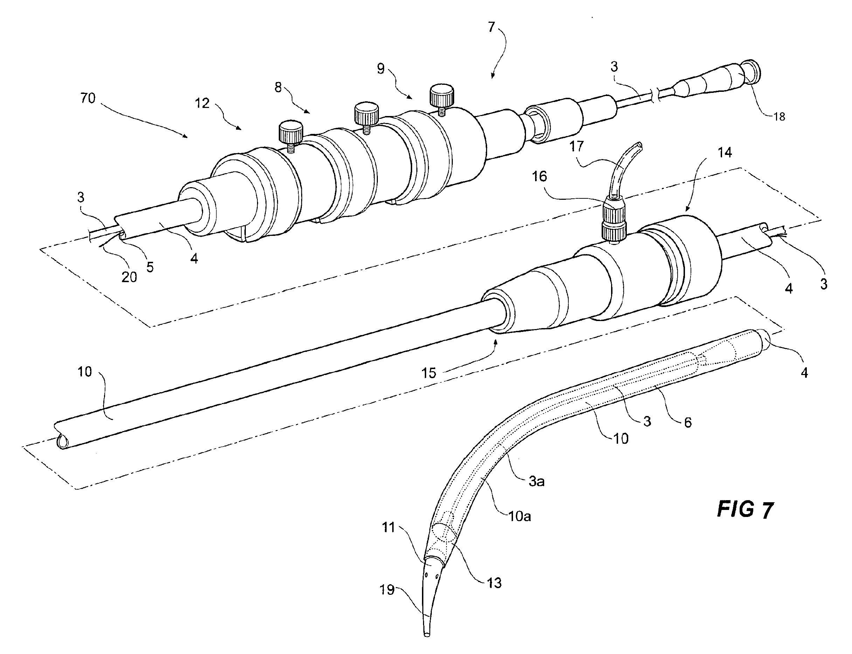

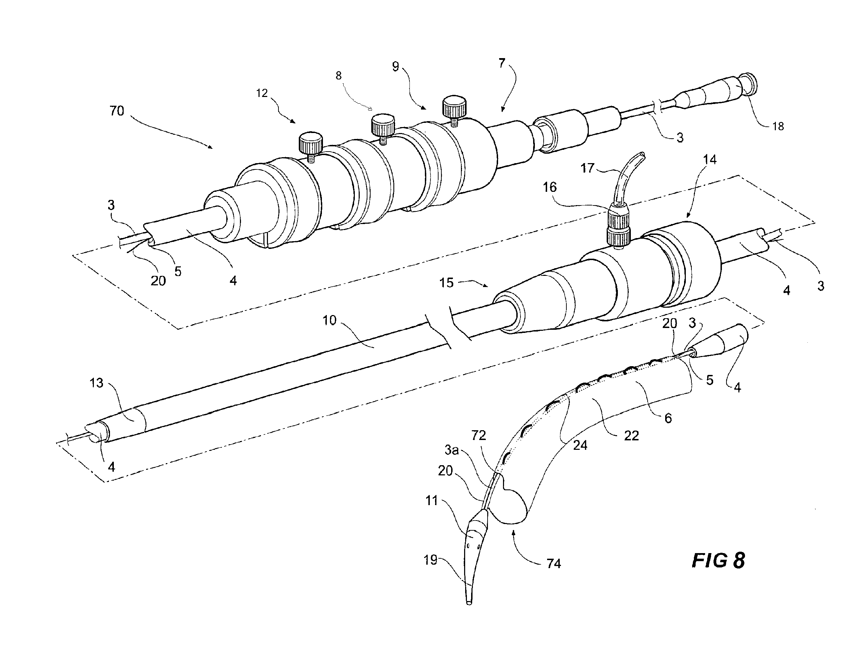

Figures **7** and **8** show an alternative embodiment. The embodiment is similar to that shown in Figure **1****,** and the same reference numerals are used for corresponding items.

The deployment device **70** has a guide wire catheter **3** which extends from a distal handle **7** to a proximal tapered nose cone dilator **11** longitudinally through a passageway or lumen **5** of a delivery catheter **4** which is connected to the handle **7** at its distal end. An introducer sheath **10** fits coaxially around the delivery catheter **4** and extends from a tapered proximal end **13** which optionally includes a radiopaque marker to a connector valve and manipulator **14** attached about distal end **15** of the sheath. The introducer sheath **10** extends proximally to the nose cone dilator **11** and covers the stent graft **6** during introduction of the deployment device into a patient. The introducer sheath **10** is withdrawn distally to expose the stent graft **6** during deployment when the deployment device is in a selected position within the vasculature of a patient.

The sheath in this embodiment is flexible but has a preformed curve **10a** towards its proximal end so that the delivery device can more easily conform to the shape of the thoracic arch without putting an unacceptable stress against the wall of the thoracic arch. The preformed curved portion of the introducer sheath **10a** is sufficiently flexible, however, that when it is retracted back to the delivery catheter **4** as shown in Figure **8****,** the curved portion **10a** of the introducer sheath **10** straightens out.

The guide wire catheter **3** also has a preformed curve **3a** just distal of the nose cone dilator **11** again so that the delivery device can more easily conform to the shape of the thoracic arch without putting an unacceptable stress against the wall of the thoracic arch.

The stent graft **6** is carried on the guide wire catheter **3** proximally of the delivery catheter **4** and distally of the nose cone dilator **11.** The stent graft **6** is not shown in Figure **7** but can be seen in Figure **8** in which the sheath **10** is shown retracted to the delivery catheter **4.** The stent graft **6** comprises a tubular body **22** of a biocompatible material and a plurality of self-expanding stents (not shown for clarity). In this embodiment, the stent graft has a scallop **72** at its proximal end **74.** When correctly deployed, the scallop will allow access to the left subclavian artery from the thoracic arc while still allowing a sufficient sealing region or landing zone between the thoracic arch and the aneurysm.

Connector valve **14** includes a silicone disk (not shown) for preventing the backflow of fluids therethrough. The disk includes a slit for the insertion of the nose cone dilator **11** and delivery catheter **4.** Connector **14** also includes side arm **16** to which a tube **17** is connected for introducing and aspirating fluids therethrough. Nose cone dilator **11** includes a tapered proximal end **19** for accessing and dilating a vascular access site over a well-known and commercially available wire guide (not shown).

To deploy the stent graft, a wire guide is inserted in the vessel with an introducer needle using, for example, a percutaneous vascular access Seldinger technique. A male Luer lock connector hub **18** is attached at the distal end of the guide wire catheter **3** for connection to syringes and other medical apparatus. The handle **7** at the distal end of the delivery catheter **4** remains outside a patient in use and carries the trigger wire release handle mechanisms **8, 9,** and **12** used to release the various portions of the stent graft **6.** The proximal end the stent graft **6** is retained on the delivery device by the use of trigger wires (not shown) connected to the release handle **9.** The distal end of the stent graft is retained on the delivery device by the use of trigger wires (not shown) connected to the release handle **8.** The handle also includes a release mechanism **12** for a release wire for the longitudinal portion of the stent graft as discussed below.

A cannula attachment wire **20** extends from the release mechanism **12** on the handle **7** through the lumen **5** in the delivery catheter **4** and exits at the proximal end of the delivery catheter **4** as shown in Figure **2****.** The release wire **20** is threaded through the guide wire catheter **3** via openings **28** and through the material of the tubular body **22** in a spiral fashion so that there are lengths **24** of release wire **20** outside the tubular body and lengths **26** of release wire **20** inside the guide wire catheter **3.** This arrangement holds the guide wire catheter **3** against a selected longitudinal portion of the stent graft.

When the stent graft is deployed into the vasculature of a patient and the device is deployed into a curved vessel the curved guide wire catheter **3a** of the deployment device will take up a position in the greater radius side of the curve. Hence the longitudinal portion of the stent graft which is releasably bound to the guide wire catheter will also take up a position at the greater radius side of the curve. This means that a stent graft with a fenestration or scallop which is to be deployed on the greater radius side of the curve can be mounted onto the deployment device with the fenestration or scallop adjacent the guide wire and once deployed the scallop or fenestration will be easier to correctly position. When it is desired to release the stent graft, the cannula attachment wire **20** can be withdrawn by removal of the release wire mechanism **12** which will release the stent graft from its engagement with the guide wire catheter.

In one aspect, methods of manufacturing an endovascular delivery device are disclosed. The method includes (a) providing a guide wire having inner and outer surfaces, the inner surface defining a passageway with a longitudinal axis, and the outer surface having a plurality of openings radially located from the longitudinal axis; (b) providing a stent graft having a tubular body made of a biocompatible material with a lumen therethrough with first and second ends and a plurality of stents, the stent graft being mounted onto the delivery device for deployment therefrom and being positioned on the guide wire catheter wherein the guide wire catheter passes through the lumen of the stent graft; (c) providing a cannula attachment wire that weaves in and out of the plurality of openings.

In various manufacturing method embodiments, the delivery device can be adapted and provided with the features as described above for the various physical embodiments.

Throughout this specification, various indications have been given as to the scope of this invention, but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

## Claims

1. An endovascular delivery device, comprising:
a nose cone dilator,
a guide wire catheter comprising:
a passageway with a longitudinal axis,
a plurality of openings radially located from the longitudinal axis;
a stent graft comprising:
a tubular body of a biocompatible material with a lumen therethrough having first and second ends,
a plurality of stents,
wherein the stent graft is operably connected to the delivery device for deployment therefrom and being positioned on the guide wire catheter, wherein the guide wire catheter passes through the lumen of the stent graft; a cannula attachment wire operably connected to guide wire catheter and stent graft, wherein the cannula attachment wire weaves in and out of the plurality of openings.

2. The endovascular delivery device of claim 1, the guide wire catheter having inner and outer surfaces, the inner surface defining a passageway with a longitudinal axis, and the outer surface having said plurality of openings radially located from the longitudinal axis;
the stent graft being mounted onto the delivery device for deployment therefrom whereby the stent graft is temporarily affixed to the guide wire catheter along a length of the guide wire catheter defined by the length of the stent graft.

3. The endovascular delivery device of claim 1 or claim 2, wherein the guide wire catheter comprises at least three pairs of openings, preferably including one pair located substantially away from the first and second ends of the stent graft.

4. The endovascular delivery device of any one of claims 1 -3, wherein the cannula attachment wire and guide wire catheter conform a shape of the stent graft.

5. The endovascular delivery device of any one of claims 1-4, wherein the cannula attachment wire weaves in and out of the stent graft.

6. The endovascular delivery device of any one of claims 1-5, wherein the cannula attachment wire is substantially parallel to the longitudinal axis.

7. The endovascular delivery device of any one of claims 1-6, wherein the plurality of openings are linearly arranged and substantially parallel to the longitudinal axis.

8. The endovascular delivery device of any one of claims 1-7, comprising a delivery catheter comprising a longitudinal lumen therethrough, the guide wire catheter extending through the longitudinal lumen so that the guide wire catheter is movable longitudinally and rotationally with respect to the delivery catheter.

9. The endovascular delivery device of any one of claims 1-7, comprising a delivery catheter further comprising a flexible sheath over the delivery catheter and extending to the nose cone dilator and thereby retaining the stent graft in a contracted conformation on the guide wire catheter.

10. The endovascular delivery device of claim 9, wherein the flexible sheath has a pre-formed curve located near the nose cone dilator.

11. The endovascular delivery device of any one of claims 1-10, further comprising a release mechanism operably connected to the cannula attachment wire.

12. The endovascular delivery device of any one of claims 1-11, wherein the guide wire catheter has a pre-formed curve located near the nose cone dilator.
